# EUROPEAN PATENT SPECIFICATION

(11) **EP 4 085 823 B1**
(45) Date of publication and mention of the grant of the patent: **13.08.2025**
(21) Application number: 21757294.0
(22) Date of filing: 19.02.2021
(51) Int. Cl.: A61B 5/00, A61B 5/145, A61B 5/15, A61B 5/155, H04L 1/16

(54) **METHOD FOR TRANSMITTING AND RECEIVING NON-RECEIVED BIOMETRIC INFORMATION ON BASIS OF DOMAIN TO WHICH NON-RECEIVED BIOMETRIC INFORMATION BELONGS**
VERFAHREN ZUM SENDEN UND EMPFANGEN VON NICHT EMPFANGENEN BIOMETRISCHEN INFORMATIONEN AUF DER BASIS EINER DOMÄNE, ZU DER NICHT EMPFANGENE BIOMETRISCHE INFORMATIONEN GEHÖREN
PROCÉDÉ D'ÉMISSION ET DE RÉCEPTION D'INFORMATIONS BIOMÉTRIQUES NON REÇUES SUR LA BASE D'UN DOMAINE AUQUEL APPARTIENNENT DES INFORMATIONS BIOMÉTRIQUES NON REÇUES

(30) Priority: 19.02.2020 KR 20200020123
(43) Date of publication of application: 09.11.2022
(73) Proprietor: i-Sens, Inc., Seoul 06646 (KR)
(72) Inventor: YOU, Choong Beom, Seoul 06646 (KR); LEE, Ye Jeong, Seoul 06646 (KR); HEO, Hun Woo, Seoul 06646 (KR); SEO, A Ri, Seoul 06646 (KR)
(74) Representative: BCKIP Part mbB
(86) International application number: PCT/KR2021/002092
(87) International publication number: WO 2021/167384

(56) References cited:
- EP-A1- 3 135 195
- EP-A1- 3 984 447
- EP-B1- 3 113 054
- WO-A1-2017/108996
- WO-A1-2021/015389
- AU-A1- 2016 219 530
- JP-A- 2012 064 019
- JP-A- 2012 064 019
- JP-A- 2019 510 388
- KR-A- 20070 031 810
- KR-A- 20110 121 445
- US-A1- 2015 359 490
- US-A1- 2016 174 853
- US-A1- 2016 335 409
- US-A1- 2017 156 682

## Description

### TECHNICAL FIELD

The present invention generally relates to a computer readable medium containing a program, which, when executed by a computer, causes the computer to carry out a method for transmitting and receiving unreceived biometric information, and more specifically, a method for transmitting and receiving unreceived biometric information, the method of, when unreceived biometric information exists, differently controlling receipt of the unreceived biometric information by determining whether the unreceived biometric information belongs to a dangerous range or a normal range based on received adjacent biometric information.

### BACKGROUND

Diabetes is a chronic medical condition that is common in modern people, and in the Republic of Korea, there are 2 million diabetes patients, about 5% of the total population.

Diabetes occurs when the absolute level of the sugar level in blood is high due to the absolute deficiency or relative insufficiency of insulin, produced by the pancreas, caused by various reasons such as obesity, stress, poor eating habits, and inherited hereditary factors and imbalance regarding glucose in the blood.

The blood usually contains a certain concentration of glucose, and tissue cells gain energy from the glucose.

However, when the glucose is increased excessively more than needed, the glucose cannot be properly stored in the liver, muscle, or adipose tissue and is accumulated in the blood, because of this, patients with diabetes maintain a much higher blood glucose level than normal people, and as excessive blood glucose passes through the tissues and is discharged into the urine, it results in deficiency of glucose, which is absolutely necessary for all tissues of the body, thereby causing abnormalities in respective body tissues.

Diabetes is characterized by substantial absence of subjective symptoms at the beginning of the condition, when diabetes progresses, diabetes-specific symptoms such as overdrink, overeat, polyuria, weight loss, weariness, skin itchiness, and lower ability of naturally healing on injury on hands and feet are shown, and further progression of diabetes leads to complications such as visual disturbances, hypertension, kidney disease, paralysis, periodontal disease, muscle spasms and neuralgia, as well as gangrene.

In order to diagnose diabetes beforehand and manage to prevent the progression of diabetes into complications associated therewith, systematic blood glucose measurement and treatment should be performed.

Diabetes need to constantly measure blood glucose for management, so the demand for devices related to blood glucose measurement is steadily increasing. It has been confirmed through various studies that, when diabetic patients strictly control the management of blood glucose, the incidence of complications of diabetes is significantly reduced. Accordingly, it is very important for diabetic patients to measure blood glucose regularly for blood glocuse management.

In general, a finger prick type method is mainly used for blood glucose control in diabetic patients. This blood prick type method helps diabetic patients to manage their blood glucose, but because only the result at the time of measurement is displayed, there is a problem that it is difficult of precisely monitoring the blood glucose level that changes frequently. **In** addition, since the blood prick type blood glucose meter needs to collect blood every time to measure blood glucose frequently during the day, there is a problem in that the burden of blood collection is huge for diabetic patients.

Diabetics patients generally experience hyperglycemia and hypoglycemia, and an emergency may occur in the hypoglycemic conditions. Hypoglycemia occurs when sugar content is not kept for a long time, and the patients may become unconscious or die in a worst case. Accordingly, rapid discovery of the hypoglycemic condition is critically important for diabetics. The figure prick type blood glucose meter intermittently measuring glucose have limited ability to accurately measure blood glucose levels.

Recently, to overcome such a drawback, continuous glucose monitoring systems (CGMSs) inserted into the human body to measure a blood glucose level every few minutes have been developed, and therefore easily perform the management of diabetics and responses to an emergency situation.

The continuous glucose monitoring system includes a sensor transmitter configured to be attachable to a body part of a user and measure blood glucose by extracting body fluid, a communication terminal configured to output the received blood glucose level, and so on. The sensor transmitter measures the blood glucose of the user in a status that a sensor is inserted to a human body for a certain period, for example, fifteen (15) days, and generates blood glucose information. The sensor transmitter periodically generates blood glucose information, and the communication terminal periodically receives and outputs the blood glucose information so that the user can check the received blood glucose information.

In the continuous blood glucose measurement system described above, the sensor transmitter and the communication terminal transmit and receive blood glucose information in a wired communication type or a wireless communication type, and the communication terminal must continuously receive transmission packets from the sensor transmitter.

However, it may occur that the communication terminal cannot continuously receive blood glucose information from the sensor transmitter due to temporary communication disconnection between the sensor transmitter and the communication terminal or the user's inexperienced operation, or when the communication terminal and the sensor transmitter are disposed away from each other by a distance that cannot communicate with each other for a considerable period of time, the communication terminal may not be able to receive the user's blood glucose information during the corresponding time period.

As such, when the communication terminal is unable to receive blood glucose information from the sensor transmitter, the communication terminal needs to inform the user that unreceived blood glucose information exists and receive the unreceived blood glucose information, but, if repeatedly notifying the user that the unreceived blood glucose information exists even when many notifications already have been repeatedly sent to the user or unreceived blood glucose information is unnecessary to the user, it may cause inconvenience to the user.

US 2016 / 0 335 409 A1 discloses techniques for receiving glucose data from a continuous glucose sensor and controlling the use and redistribution of that data so it is used in an intended manner. A method includes obtaining one or more data points relating to glucose levels from a transmitter associated with a continuous glucose monitor device; distributing the one or more data points among one or more display devices and one or more servers; identifying a missing data point from among a display device of the one or more display devices or a server of the one or more servers, the missing data point being one of the one or more data points; and providing the missing data point to the display device or the server when the missing data point falls within a defined time period.

### DISCLOSURE OF THE INVENTION

### Technical Problem

To solve the problem of the conventional method of transmitting and receiving biometric information described above, the purpose of the present invention is to provide a method for transmitting and receiving biometric information, the method determining whether biometric information unreceived from a sensor transmitter by a communication terminal exists, and, when the unreceived biometric information exists, outputting an indicator of the unreceived biometric information to a user to induce to receive the unreceived biometric information through the indicator.

Another purpose of the present invention is for providing a method for transmitting and receiving biometric information, the method of, when unreceived biometric information exists, differently controlling receipt of the unreceived biometric information by determining whether the unreceived biometric information belongs to a dangerous range or a normal range based on received adjacent biometric information.

Still another purpose of the present invention is for providing a method for transmitting and receiving biometric information, the method of, when biometric information unreceived from a sensor transmitter by a communication terminal exists and the unreceived biometric information belongs to a normal range, processing to make an indicator notifying the existence of the unreceived biometric information being blinded or removing the indicator after a certain time elapses in order to reduce the inconvenience of the user caused by frequent notification of unnecessary unreceived biometric information.

Still another purpose of the present invention is for providing a method for transmitting and receiving biometric information, the method of, when biometric information unreceived from a sensor transmitter by a communication terminal belongs to a dangerous range and as a result the unreceived biometric information is received, determining whether the unreceived biometric information belongs to an actual dangerous range and informing the user that the unreceived biometric information belongs to the actual dangerous range.

### Solution to Problem

To accomplish the purpose of the present invention, the present invention provides a computer readable medium in accordance with claim 1.

According to the present invention, a computer readable medium containing a program, which, when executed by a computer, causes the computer to carry out a method for transmitting and receiving biometric information, wherein the method comprises: determining whether unreceived biometric information among biometric information measured by the sensor transmitter exists; when the unreceived biometric information exists, outputting an indicator for existence of the unreceived biometric information to be informed to the user; and requesting and receiving the unreceived biometric information from the sensor transmitter.

According to the present invention, the method for transmitting and receiving biometric information further comprises determining whether the unreceived biometric information belongs to a dangerous range or a normal range.

According to the present invention, in the method for transmitting and receiving biometric information, information on a range to which the unreceived biometric information belongs is included in the indicator and outputted.

Preferably, according to an embodiment of the present invention, a method for transmitting and receiving biometric information further comprises, when the unreceived biometric information exists, determining time of measuring the unreceived biometric information, wherein a range to which the unreceived biometric information belongs is determined based on biometric information received around the time of measuring the unreceived biometric information.

Here, according to an embodiment of the present invention, in a method for transmitting and receiving biometric information, when the unreceived biometric information belongs to the normal range, the indicator is processed to be blinded so that the user is unable to visually recognize the indicator.

Here, according to an embodiment of the present invention, in a method for transmitting and receiving biometric information, wherein when the unreceived biometric information exists and the unreceived biometric information belongs to the normal range, the unreceived biometric information is requested and received from the sensor transmitter only if a receipt command for receiving the unreceived biometric information is inputted.

Here, according to an embodiment of the present invention, in a method for transmitting and receiving biometric information, when the unreceived biometric information exists and the unreceived biometric information belongs to the normal range, the unreceived biometric information is requested and received from the sensor transmitter if the receipt command for receiving the unreceived biometric information is inputted within a first threshold time, and when the receipt command is not inputted during the first threshold time, the indicator outputted on a display is deleted.

Preferably, according to an embodiment of the present invention, a method for transmitting and receiving biometric information further comprises: when the unreceived biometric information exists and the unreceived biometric information belongs to the dangerous range, determining whether the receipt command for receiving the unreceived biometric information is inputted within a second threshold time; when the receipt command for receiving the unreceived biometric information is not inputted within the second threshold time, outputting to the user an inquiry message for inquiring whether to receive the unreceived biometric information; and determining whether the receipt command is inputted within a third threshold time after outputting the inquiry message, wherein: when the receipt command is inputted within the third threshold time, the unreceived biometric information is requested and received from the sensor transmitter, and when the receipt command is not inputted during the third threshold time, the indicator outputted on a display is automatically deleted.

Preferably, according to an embodiment of the present invention, a method for transmitting and receiving biometric information further comprises, when the unreceived biometric information exists: determining time of measuring the unreceived biometric information; and based on the determined time of measuring the unreceived biometric information, calculating deletion time of deleting the unreceived biometric information of which storage time set after measuring and storing the unreceived biometric information by the sensor transmitter elapses, wherein at least one of the first threshold time, the second threshold time, and the third threshold time is calculated based on the deletion time.

Here, the first threshold time, the second threshold time, and the third threshold time are calculated to be prior to the deletion time.

Preferably, according to an embodiment of the present invention, a method for transmitting and receiving biometric information further comprises: when the unreceived biometric information belongs to the dangerous range, receiving the unreceived biometric information from the sensor transmitter and determining whether the unreceived biometric information belongs to an actual dangerous range; and when it is determined that the unreceived biometric information belongs to the actual dangerous range, generating and outputting an alarm message notifying the dangerous range to the user.

### Advantageous Effects of the Invention

A method for transmitting and receiving unreceived biometric information according to the present invention has the following effects.

First, the method for transmitting and receiving biometric information determines whether biometric information unreceived from a sensor transmitter by a communication terminal exists, and, when the unreceived biometric information exists, outputs an indicator of the unreceived biometric information to a user to induce to receive the unreceived biometric information through the indicator.

Second, when unreceived biometric information exists, the method for transmitting and receiving biometric information differently controls receipt of the unreceived biometric information by determining whether the unreceived biometric information belongs to a dangerous range or a normal range based on received adjacent biometric information.

Third, when biometric information unreceived from a sensor transmitter by a communication terminal exists and the unreceived biometric information belongs to a normal range, a method for transmitting and receiving biometric information processes to make an indicator notifying the existence of the unreceived biometric information being blinded or removes the indicator after a certain time elapses in order to reduce the inconvenience of the user caused by frequent notification of unnecessary unreceived biometric information.

Fourth, when biometric information unreceived from a sensor transmitter by a communication terminal belongs to a dangerous range and as a result the unreceived biometric information is received, a method for transmitting and receiving biometric information determines whether the unreceived biometric information belongs to an actual dangerous range and informs the user that the unreceived biometric information belongs to the actual dangerous range, so that the user can check whether the unreceived biometric information is actually dangerous.

### BRIEF DESCRIPTION OF THE DRAWINGS

FIG. 1 is a schematic diagram for illustrating a continuous blood glucose measurement system according to an embodiment of the present invention.
FIG. 2 is a figure for illustrating an example of generating blood glucose information in a sensor transmitter.
FIG. 3 is a figure for illustrating an example of generating a transmission packet in a sensor transmitter.
FIG. 4 is a functional block diagram for illustrating a communication terminal according to an embodiment of the present invention.
FIG. 5 is a functional block diagram for illustrating a biometric information management module according to an embodiment of the present invention.
FIG. 6 is a flow chart for illustrating a method for receiving and transmitting unreceived blood glucose information according to an embodiment of the present invention.
FIG. 7 illustrates an example of an indicator according to the present invention.
FIG. 8 shows an example of a user interface screen for inputting a user command for receiving unreceived blood glucose information.
FIG. 9 is a flowchart for illustrating a method for transmitting and receiving unreceived blood glucose information based on a range to which the unreceived blood glucose information belongs according to an embodiment of the present invention.
FIG. 10 is a figure for explaining a section in which blood glucose information is not received from the sensor transmitter.
FIG. 11 is a figure for explaining an example of determining a range, to which unreceived blood glucose information belongs, from adjacent blood glucose information.
FIG. 12 is a figure for illustrating another example of determining a range, to which unreceived blood glucose information belong, from adjacent blood glucose information
FIG. 13 is a flowchart for explaining a method for transmitting and receiving unreceived blood glucose information based on a range to which the unreceived blood glucose information belongs according to an embodiment of the present invention.
FIG. 14 is a flowchart for illustrating an example of calculating a first threshold time, a second threshold time, or a third threshold time according to an embodiment of the present invention.
FIG. 15 is a figure for illustrating an example of calculating a first threshold time to a third threshold time.

### DESCRIPTION OF EMBODIMENTS OF THE INVENTION

The technical terms used in the present disclosure are only for the purpose of describing exemplary embodiments, and they are not intended to limit the present invention. Also, unless otherwise defined, all technical terms used herein should be construed as having the same meaning as commonly understood by those skilled in the art, and should not be interpreted as being excessively inclusive or excessively restrictive. In addition, when a technical term used herein is an erroneous technical term that does not accurately represent the idea of the present invention, it should be understood as replacing the term by a technical term which can be properly understood by those skilled in the art.

Further, singular expressions used in the present specification include plural expressions unless they have definitely opposite meanings. In the present application, it shall not be construed that terms, such as "including" or "comprising", various constituent elements or steps described in the specification need to be all essentially included, and it shall be construed that some constituent elements or steps among the various constituent elements or steps may be omitted, or additional constituent elements or steps may be further included.

Also, it should be noted that the accompanying drawings are merely illustrated to easily explain the invention, and therefore, they should not be construed to limit the invention by the accompanying drawings.

Hereinafter, with reference to the enclosed drawings, a method for receiving and transmitting biometric information according to an embodiment of the present invention is described in detail.

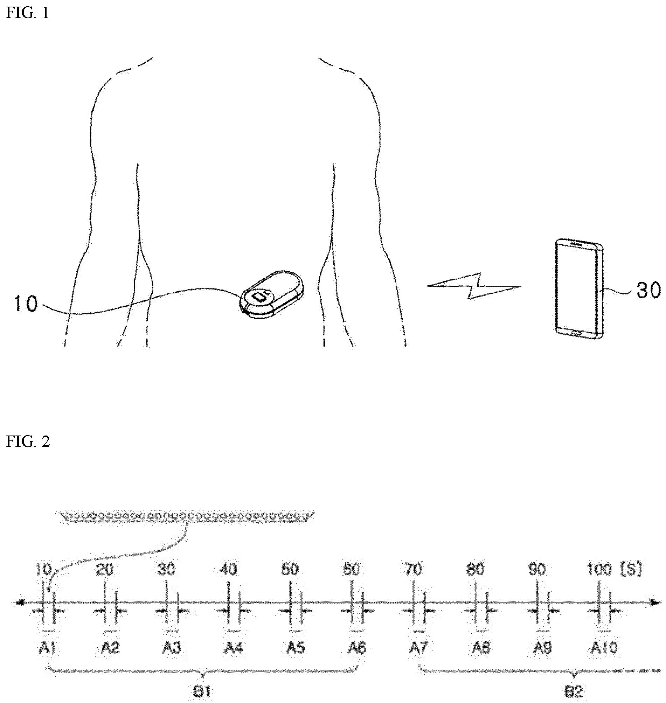

FIG. 1 is a schematic diagram for illustrating a continuous blood glucose measurement system.

Referring to FIG. 1, the continuous blood glucose measurement system (1) comprises a sensor transmitter (10) and a communication terminal (30).

The sensor transmitter (10) is attachable to human body and, when the sensor transmitter (10) is attached to the human body, an end portion of a sensor of the sensor transmitter (10) is inserted into skin to periodically extract body fluid of the human body and measure blood glucose.

The communication terminal (30) is a terminal configured to receive blood glucose information from the sensor transmitter (10) and output or display the received blood glucose information to a user, and for example, the communication terminal (30) may be a portable terminal (such as smartphone, tablet PC, or notebook and so on) configured to communicate with the sensor transmitter (10). However, the communication terminal (30) is not limited thereto, and may be any type of a terminal to which has a communication function and program or application can be installed.

The sensor transmitter (10) transmits the blood glucose information in response to request of the communication terminal (30) or at predetermined times periodically, and for data communication between the sensor transmitter (10) and the communication terminal (30), the sensor transmitter (10) and the communication terminal (30) are communicationally connected to each other over a wire by an USB cable and so on or communicationally connected in an wireless communication means such as infrared communication, NFC communication, Bluetooth, etc.

Here, the communication terminal (30) determines whether blood glucose information that has not been received from the sensor transmitter (10) exists, and if there is unreceived blood glucose information, a user interface screen for notifying an user that the blood glucose information that has not been received exists, and the communication terminal (30) receives the unreceived blood glucose information according to a selection of the user or a region to which the blood glucose information belongs.

Depending on the field to which the present invention is applied, various types of biometric information may be measured in addition to the blood glucose information, but, hereinafter, the blood glucose information will be described as an example of the biometric information.

FIG. 2 is a figure for illustrating an example of generating blood glucose information in a sensor transmitter.

First, data regarding a blood glucose signal measured by a sensor transmitter are measured at every certain time period, and, for every one time period for the measurement, multiple time measurement can be performed. For example, the sensor transmitter measures blood glucose signal data every ten (10) seconds. At that time, every time when one unit measurement is performed, blood glucose signals are measured thirty (30) times, and the time consumed for measuring the blood glucose signals may be one (1) second. Accordingly, the sensor transmitter measures thirty (30) analog blood glucose signal data every ten (10) seconds.

Accordingly, for example, blood glucose information can be measured every ten seconds, such as thirty times measurements of blood glucose information between 2 o'clock 14 minute 25 second and 2 o'clock 14 minute 26 second PM, and another thirty times measurements of blood glucose information between 2 o'clock 14 minute 35 second and 2 o'clock 14 minute 36 second PM.

The measured blood glucose signal data is transformed into a digital signal. The sensor transmitter calculates one average value every ten seconds by calculating an average value of thirty blood glucose information data transformed to the digital signal by a trimmed average calculation way. At that time, seven highest data and seven lowest data among the thirty blood glucose information data are removed and an average value (A) of remaining sixteen data is calculated.

The trimmed average value (A) calculated by way of being described above can be generated every ten (10) seconds, and, as illustrated, six (6) trimmed average values (A1 to A6) can be generated for one (1) minute.

Additionally, six trimmed average values (A1 to A6) are generated for one minute, and a second trimmed average value (B1) is generated using the generated six trimmed average values (A1 to A6). At that time, the generated second trimmed average value (B1) is calculated by removing a maximum value and a minimum value among six trimmed average values (A1 to A6) and calculating an average of remaining four values. Accordingly, blood glucose information is generated from one second trimmed average value (B) for one minute.

The blood glucose information data generated every minute is stored at the sensor transmitter, and the stored blood glucose information can be generated as a transmission packet to be transmitted to the communication terminal.

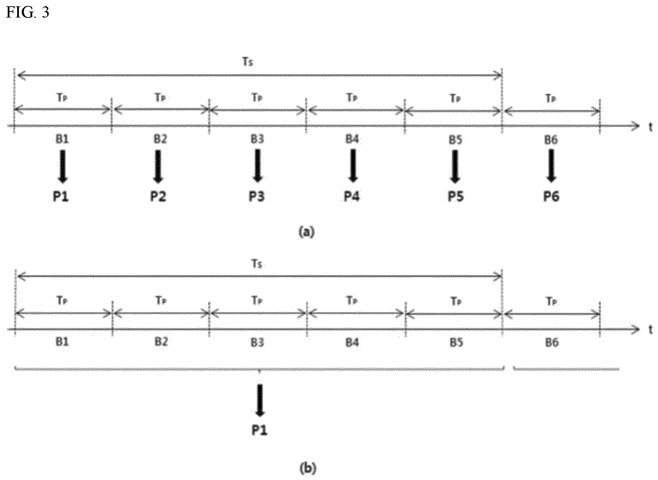

FIG. 3 is a figure for illustrating an example of generating a transmission packet in a sensor transmitter, and regarding an example of generating a transmission packet with reference to FIG. 3(a), blood glucose information (B1, B2, B3, B4, B5, B6, ...) is generated sequentially at each set blood glucose information generation period (T_{P}), and, each time the blood glucose information is generated, a transmission packet (P1, P2, P3, P4, P5, P6) having corresponding blood glucose information is generated. When the transmission packet is generated, a series of unique identifiers are allocated according to order of the generation of the transmission packets, and the transmission packet is generated to include the identifier of the transmission packet and blood glucose information. Preferably, a sequence sequentially increasing according to order of the generation of the transmission packets may be allocated as an identifier of a transmission packet, or the time of the generation of the transmission packet may be allocated as an identifier of a transmission packet.

The generated transmission packets (P1, P2, P3, P4, P5, P6) are stored in a storage module, and when a set communication period (T_{S}) is reached, the transmission packets (P1, P2, P3, P4, P5) stored in the storage module are respectively transmitted to the communication terminal.

Regarding another example of generating a transmission packet with reference to FIG. 3(b), blood glucose information (B1, B2, B3, B4, B5, B6, ...) is generated sequentially at each set blood glucose information generation period (T_{P}) and is stored in the storage module every time the blood glucose information is generated, and when a set communication period (T_{S}) is reached, a transmission packet (P1) including all blood glucose information stored in the storage module for the communication period is generated and the generated transmission packet (P1) is transmitted to the communication terminal.

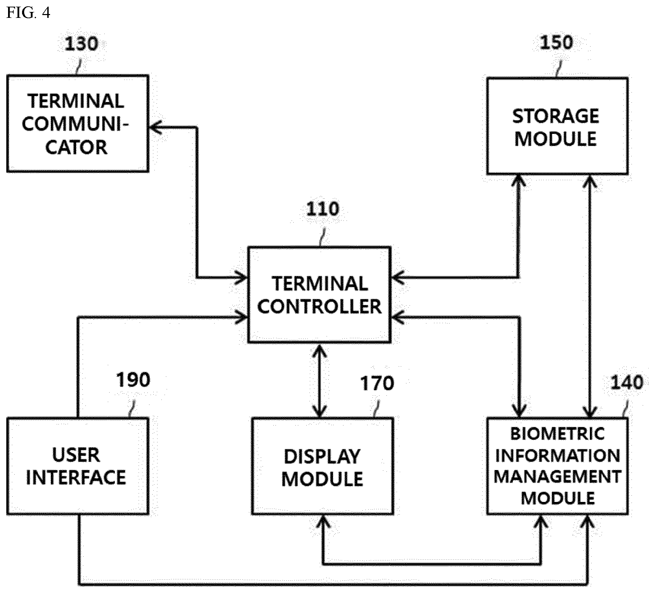

FIG. 4 is a functional block diagram for illustrating a communication terminal.

Referring to FIG. 4, when an advertisement message transmitted from a sensor transmitter at each set communication interval is received through a terminal communicator (130), a terminal controller (110) connects the communication with the sensor transmitter through the terminal communicator (130). When the terminal controller (110) is connected to the communication with the sensor transmitter, the terminal controller (110) receives blood glucose information from the sensor transmitter through the terminal communicator (130) and stores the received blood glucose information in the storage module (150). The terminal controller (110) terminates the communication with the sensor transmitter when the communication interval elapses, or terminates the communication with the sensor transmitter when the receipt of the transmission packet from the sensor transmitter is completed even before the communication interval elapses.

Meanwhile, a biometric information management module (140) determines whether unreceived blood glucose information exists based on the blood glucose information stored in the storage module (150), and if there is unreceived blood glucose information, the biometric information management module (140) outputs, to an user through a display module (170), information such as information regarding whether the unreceived blood glucose information exists, information regarding a range to which the unreceived blood glucose information belongs (information regarding whether the unreceived blood glucose information is within a dangerous range or a normal range), and the number of the unreceived blood glucose information.

The biometric information management module (140) generates an indicator including information such as information regarding whether unreceived blood glucose information exists, information regarding a range to which the unreceived blood glucose information belongs, the number of the unreceived blood glucose information and so on, and the biometric information management module (140) displays information on the generated indicator on the display module (170) to inform the user that the unreceived blood glucose information exists. When a user command for receiving the unreceived blood glucose information is inputted from a user interface (190), the transmission of the unreceived blood glucose information is requested to the sensor transmitter according to the user command to receive the unreceived blood glucose information.

The communication terminal is communicatively connected to the sensor transmitter at every set period to receive blood glucose information from the sensor transmitter, and, preferably, according to an embodiment of the present invention, the biometric information management module (140) can determine whether unreceived blood glucose information exists at current time based on the last receipt time or the last measurement time of the blood glucose information stored in the storage module (150).

Preferably, according to another embodiment of the present invention, when the biometric information management module (140) confirms communication connection with the sensor transmitter through the terminal controller (110), the biometric information management module (140) can determine whether unreceived blood glucose information exists at current time based on the last receipt time or the last measurement time of the blood glucose information stored in the storage module (150).

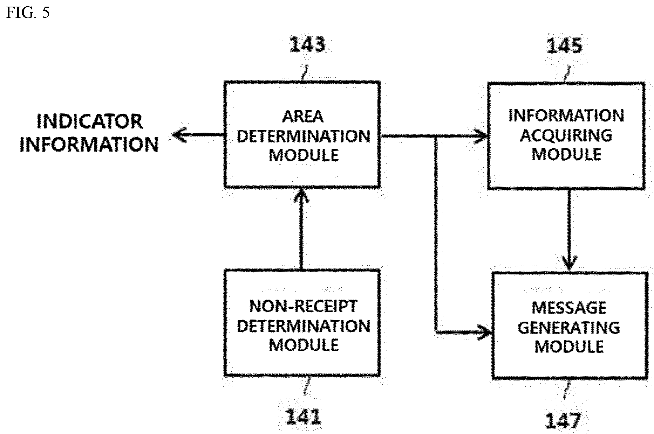

FIG. 5 is a functional block diagram for illustrating a biometric information management module.

Referring to FIG. 5, a non-receipt determination module (141) determines whether unreceived blood glucose information exists based on the blood glucose information received from the sensor transmitter and stored in the storage module. Here, an example of determining whether blood glucose information unreceived by the non-receipt determination module (141) exists is that, when the communication between the communication terminal and the sensor transmitter is connected, whether there is blood glucose information which the communication terminal has not received can be determined based on a blood glucose information identifier received from the sensor transmitter and an identifier of blood glucose information stored last in the storage module. By determining whether there is unreceived blood glucose information when the communication is connected between the communication terminal and the sensor transmitter, unnecessary operation for determining whether unreceived blood glucose information exists or unnecessary notification to an user that there is unreceived blood glucose information can be controlled to be prevented when the communication connection between the communication terminal and the sensor transmitter is impossible or when the user intentionally blocks the communication connection between the communication terminal and the sensor transmitter.

Here, another example of determining whether blood glucose information unreceived by the non-receipt determination module (141) exists is that, when new blood glucose information has not been stored to the storage module for a set threshold time from the time when last blood glucose information was stored to the storage module of the communication terminal, unreceived blood glucose information exists. Like this, by determining that there is unreceived blood glucose information when new blood glucose information has not been received continuously for the threshold time after last blood glucose information was stored to the storage module, the communication connection between the communication terminal and the sensor transmitter can be induced to receive the unreceived blood glucose information when the communication connection between the communication terminal and the sensor transmitter cannot be performed for long time.

When unreceived blood glucose information exists, an area determination module (143) determines a range to which the unreceived blood glucose information belongs. Accordingly, the area determination module (140) determines whether a range to which the unreceived blood glucose information belongs is in a dangerous range or in a normal range, and the dangerous range may mean a hyperglycemia or hypoglycemia area set by the user and the normal range may mean a normal range of a blood glucose area set by the user. The area determination module (140) generates indicator information regarding unreceived blood glucose information based on the area to which the unreceived blood glucose information belongs and controls to output it to the display module.

An information acquiring module (145) performs operations differently depending on whether a range to which the unreceived blood glucose information belongs is in the dangerous range or in the normal range, when the unreceived blood glucose information belongs to the normal range the information acquiring module (145) notifies existence of the unreceived blood glucose information one time only thereby reducing inconvenience of the user caused by frequent alarms or performs an operation that the indicator is blinded after certain time period elapses, and when the unreceived blood glucose information belongs to the dangerous range the alarms are provided to the user more frequently than the unreceived blood glucose information belonging to the normal range thereby inducing to receive the unreceived blood glucose information. When a user command for receiving the unreceived blood glucose information is inputted through a user interface module, the information acquiring module (145) receives the unreceived blood glucose information from the sensor transmitter and controls to store the received blood glucose information to the storage module.

On the other hand, when determining that unreceived blood glucose information belongs to the dangerous range and the unreceived blood glucose information received according to the user request is in an actual dangerous range, a message generating module (147) generates a message informing that the unreceived blood glucose information is in the actual dangerous range and controls to display it to the user through the display module.

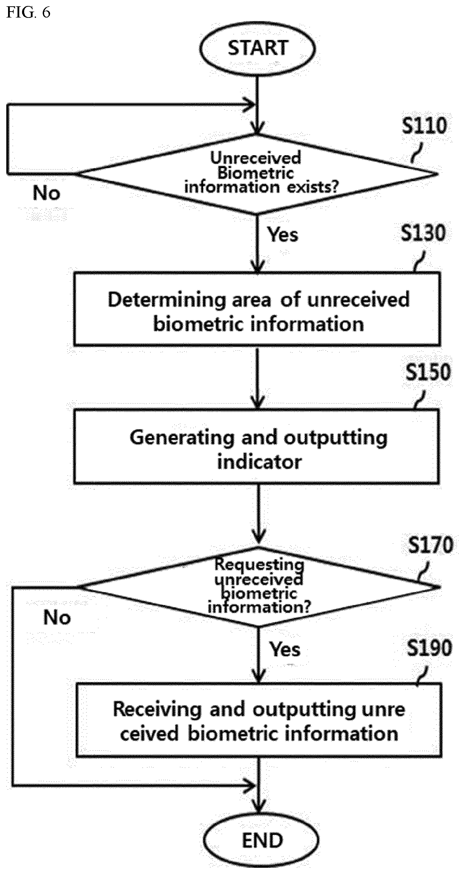

FIG. 6 is a flow chart for illustrating a method for receiving and transmitting unreceived blood glucose information according to an embodiment of the present invention.

Referring to FIG. 6, whether blood glucose information not received by the communication terminal from the sensor transmitter exists is determined (S110).

When the unreceived blood glucose information exists, time of measuring the unreceived blood glucose information is predicted and a range to which the unreceived blood glucose information belongs is determined based on blood glucose information received around the time of measuring the unreceived blood glucose information (S130).

An indicator including a range to which unreceived blood glucose information belongs, the number of the unreceived blood glucose information and so on is generated and the generated indicator is displayed on the display module (S150).

Whether a user command for requesting unreceived blood glucose information is inputted is determined based on the displayed indicator (S170), and when the user command for requesting unreceived blood glucose information is inputted, the unreceived blood glucose information is received by requesting to receive the unreceived blood glucose information from the sensor transmitter and the received unreceived blood glucose information is displayed on the display module (S190).

Preferably, sequence of the unreceived blood glucose information or an identifier of measurement time of the unreceived blood glucose information and so on are received from the sensor transmitter and the unreceived blood glucose information is requested, and the sensor transmitter searches for unreceived blood glucose information which the communication terminal has not received and provides the unreceived blood glucose information to the communication terminal.

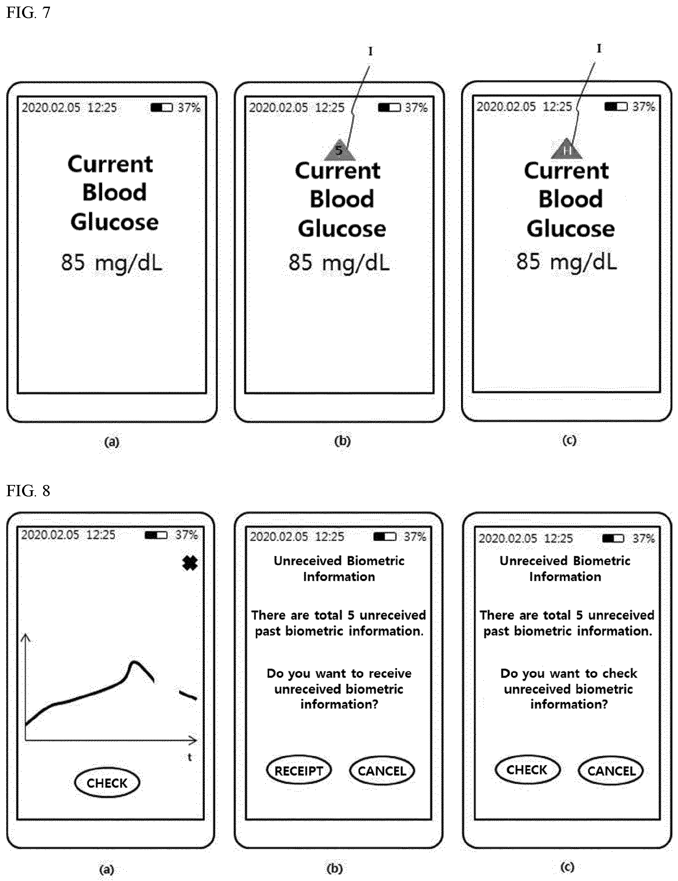

FIG. 7 illustrates an example of an indicator according to the present invention, and, as illustrated in FIG. 7(a), when there is no unreceived blood glucose information, a separate indicator is not activated on a display module of the communication terminal.

However, when unreceived blood glucose information exists, an indicator is activated as illustrated in FIG. 7(b) or 7(c), and, as illustrated in FIG. 7(b), when there is unreceived blood glucose information, the unreceived blood glucose information belongs to the normal range, the number of the unreceived blood glucose information is five (5), an identifier (I) including a range to which the unreceived blood glucose information belongs and the number of the unreceived blood glucose information is activated and, as illustrated in FIG. 7(c), when there is unreceived blood glucose information and the unreceived blood glucose information belongs to a hyperglycemia dangerous range, an identifier (I) including a range to which the unreceived blood glucose information belongs is activated.

Preferably, indicators for notifying unreceived blood glucose information belonging to the normal range and unreceived blood glucose information belonging to the dangerous range are displayed in different colors from each other, so that the user intuitively can recognize that there is unreceived blood glucose information through the indicator and can easily and quickly recognize a range to which the unreceived blood glucose information belongs.

FIG. 8 shows an example of a user interface screen for inputting a user command for receiving unreceived blood glucose information, and, when a user touches an indicator icon, received blood glucose information is displayed on a time axis of a graph as shown in FIG. 8(a) and the unreceived blood glucose information is indicated on the graph. After the user visually checks the time of measuring the unreceived blood glucose information or a range to which the unreceived blood glucose information belongs through the graph, the user can click the CHECK button and a user interface for inquiring whether to receive the unreceived blood glucose information is displayed as shown in FIG. 8(b), and, when a user command for a request for receipt is inputted by clicking the RECEIPT button on the user interface screen displayed in FIG. 8(b), unreceived blood glucose information is received. As shown in FIG. 8(c), when the unreceived blood glucose information is received, a user interface screen for inquiring about whether to check the received blood glucose information is activated.

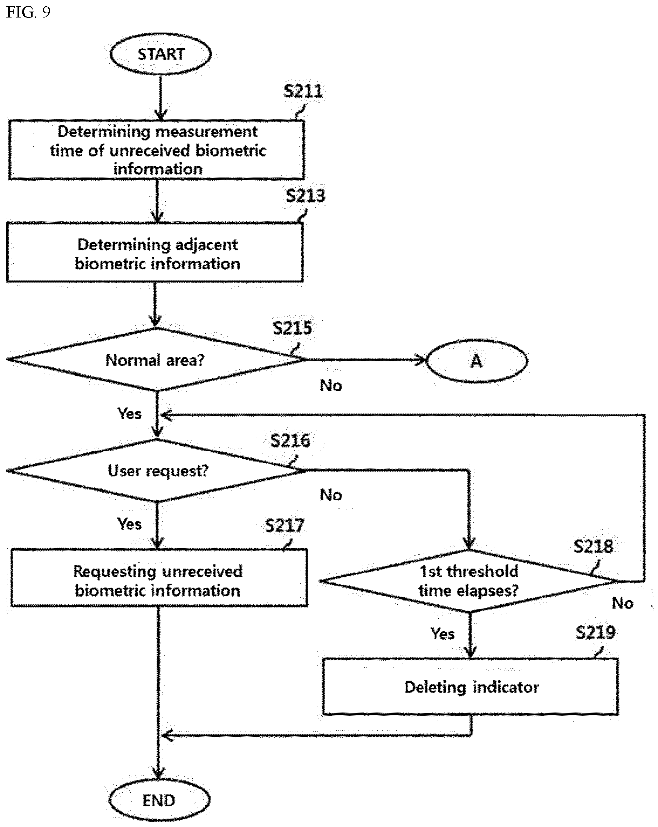

FIG. 9 is a flowchart for illustrating a method for transmitting and receiving unreceived blood glucose information based on a range to which the unreceived blood glucose information belongs according to the present invention.

Referring to FIG. 9, when unreceived blood glucose information exists, the time of measuring the unreceived blood glucose information is predicted and determined (S211). The sensor transmitter measures blood glucose information at a set cycle, and, for example, the time of measuring the unreceived blood glucose information may be predicted and determined based on the time of measuring other blood glucose information which is received from the sensor transmitter and stored. As another example, the time of measuring the unreceived blood glucose information may be predicted and determined based on the time at which the blood glucose information is not stored to the communication terminal.

Adjacent blood glucose information at time adjacent to the unreceived blood glucose information is determined based on the time of measuring the unreceived blood glucose information (S213), and whether the unreceived blood glucose information belongs to the normal region is determined based on the adjacent blood glucose information (S215).

If the unreceived blood glucose information belongs to the normal range, an indicator for the unreceived blood glucose information is outputted and whether a user command for requesting receipt of the unreceived blood glucose information from the user is inputted is determined in response to the indicator (S216). When the user command is inputted, the unreceived blood glucose information is requested to the sensor transmitter and received from the sensor transmitter (S217). Here, when the unreceived blood glucose information is requested, an identifier of the unreceived blood glucose information (for example, a sequence of the unreceived blood glucose information, a generation time, a measurement time, etc.) may be included in the request.

Meanwhile, whether the unreceived blood glucose information belongs to the normal range and whether a user command for requesting the unreceived blood glucose information is inputted within a first threshold time are determined (S218). When the user command for requesting the unreceived blood glucose information is not inputted until the first threshold time elapses, the indicator displayed on the display module is deleted or blinded (S219).

Accordingly, when the unreceived blood glucose information belongs to the normal range and the user command for receiving the unreceived blood glucose information is not inputted within the first threshold time, an indicator indicating the existence of the unreceived blood glucose information is deleted or blinded to be easily processed without cumbersome operation or intervention of the user for processing unnecessary unreceived blood glucose information.

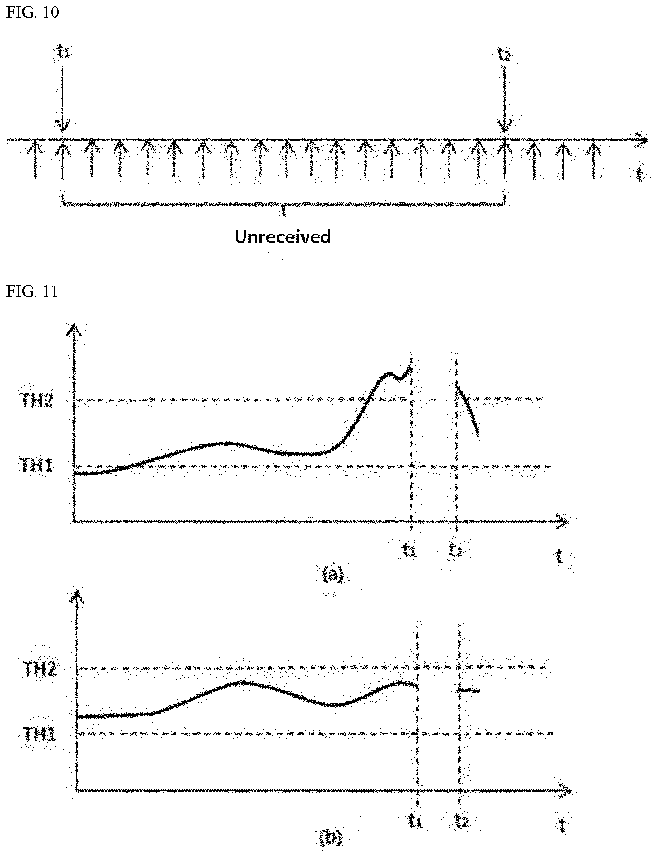

FIG. 10 is a figure for explaining a section in which blood glucose information is not received from the sensor transmitter, and, as illustrated in FIG. 10, the sensor transmitter transmits blood glucose information, generated at certain time intervals, to the communication terminal. However, the blood glucose information generated from the sensor transmitter is not received from a time point (t1) when communication between the sensor transmitter and the communication terminal is not connected to a time point (t2) when the communication is reconnected (t2).

FIG. 11 is a figure for explaining an example of determining a range, to which unreceived blood glucose information belongs, from adjacent blood glucose information, and, as illustrated in FIG. 11(a), the communication terminal has not been received blood glucose information from a first time point (t1) and starts to receive blood glucose information again from a second time point (t2). The communication terminal checks adjacent biometric information at adjacent time before the unreceived first time point (t1) and adjacent biometric information at adjacent time after the second time point (t2) based on measurement time or receipt time of blood glucose information stored in the storage module. When a range to which the adjacent biometric information at adjacent time before the unreceived first time point (t1) belongs is the dangerous range (a range higher than TH2) and a range to which the adjacent biometric information at adjacent time after the second time point (t2) belongs is the dangerous range (a range higher than TH2), it may be determined that the unreceived blood glucose information also belongs to the dangerous range.

On the other hand, as shown in FIG. 11(b), the communication terminal does not receive the blood glucose information from the first time point (t1) and starts to receive the blood glucose information again from the second time point (t2), and, when a range to which the adjacent biometric information at adjacent time before the unreceived first time point (t1) belongs is the normal range (a range lower than TH2 and higher than TH1) and a range to which the adjacent biometric information at adjacent time after the second time point (t2) belongs is the normal range (a range lower than TH2 and higher than TH1), the communication terminal may determine that the unreceived blood glucose information also belongs to the normal range.

Therefore, in FIG. 11, an example of determining a range to which the unreceived blood glucose information belongs determines a range to which the unreceived blood glucose information belongs based on other blood glucose information received at time adjacent to time before and after not receiving the unreceived blood glucose information.

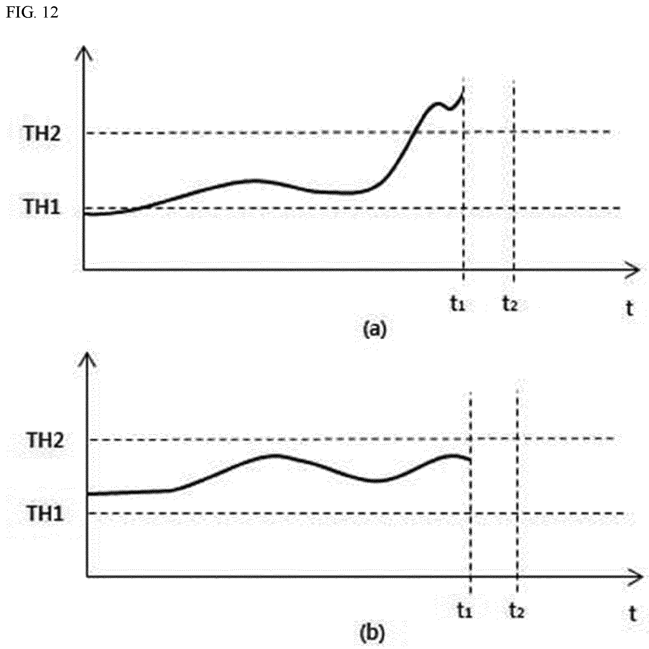

FIG. 12 is a figure for illustrating another example of determining a range, to which unreceived blood glucose information belong, from adjacent blood glucose information, and, as shown in FIG. 12(a), the communication terminal has not been received blood glucose information from a first time point (t1) and starts to receive blood glucose information again from a second time point (t2). The communication terminal checks adjacent biometric information at adjacent time before the first time point (t1) based on measurement time or receipt time of blood glucose information stored in the storage module. When a range to which the adjacent biometric information at adjacent time before the first time point (t1) belongs is the dangerous range (a range higher than TH2), it may be determined that the unreceived blood glucose information also belongs to the dangerous range.

On the other hand, as illustrated in FIG. 12(b), the communication terminal has not been received blood glucose information from the first time point (t1) and starts to receive blood glucose information again from the second time point (t2), and, when a range to which the adjacent biometric information at adjacent time before the first time point (t1) belongs is the normal range (a range lower than TH2 and higher than TH1), the communication terminal may determine that the unreceived blood glucose information also belongs to the normal range.

Therefore, in FIG. 12, an example of determining a range to which the unreceived blood glucose information belongs determines a range to which the unreceived blood glucose information belongs based on other blood glucose information received at time adjacent to time before not receiving the unreceived blood glucose information.

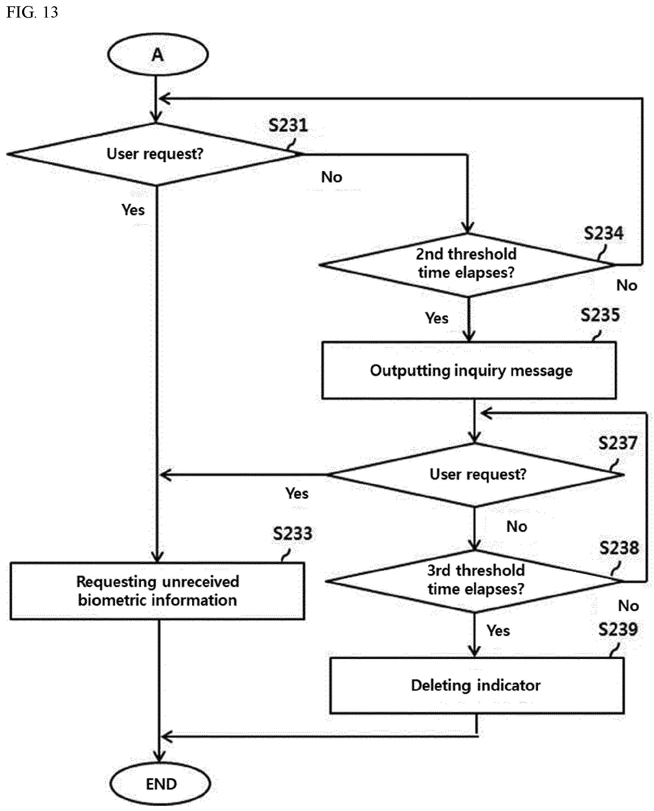

FIG. 13 is a flowchart for explaining a method for transmitting and receiving unreceived blood glucose information based on a range to which the unreceived blood glucose information belongs according to an embodiment of the present invention.

Continuing to FIG. 9, when a range to which the unreceived blood glucose information belongs is determined to be a dangerous range rather than a normal range based on adjacent blood glucose information, whether a user command for requesting the unreceived blood glucose information is inputted is determined (S231). Here, information on whether the unreceived blood glucose information belongs to the dangerous range is indicated on the indicator.

When the user command for requesting the unreceived blood sugar information is inputted, the unreceived blood glucose information is requested to the sensor transmitter and received from the sensor transmitter (S233).

However, if the user command for requesting the unreceived blood glucose information is not inputted, whether a second threshold time elapses after displaying the indicator is determined (S234). When the second threshold time elapses, an inquiry message notifying the user that because the unreceived blood glucose information exists and the unreceived blood glucose information belongs to the dangerous range it is necessary to receive and check the unreceived blood glucose information is generated and outputted (S235).

After outputting the inquiry message, whether a user command for requesting the unreceived blood glucose information is inputted is determined again (S237), and, when the user command for requesting the unreceived blood glucose information is inputted, the unreceived blood glucose information is requested to the sensor transmitter and received from the sensor transmitter.

However, whether a third threshold time has elapsed in a state in which the user command is not inputted after the inquiry message is outputted is determined (S238), and, if the user command has not been inputted for the third threshold time, it is determined that the user does not intend to receive the unreceived blood glucose information and an indicator indicating that the unreceived blood glucose information exists is deleted even though the unreceived blood glucose information belongs to the dangerous range (S239).

According to fields to which an embodiment of the present invention is applied, the dangerous range can be subdivided into a high-dangerous range and a low-dangerous range, and, when a range to which the unreceived blood glucose information belongs is determined to be the high-dangerous range based on the adjacent blood glucose information, the operation of requesting the unreceived blood glucose information to the sensor transmitter and receiving the unreceived blood glucose information from the sensor transmitter can be controlled to be performed automatically regardless of whether the user command for requesting the unreceived blood glucose information is inputted.

On the other hand, depending on fields to which an embodiment of the present invention is applied, the number or frequency of outputting inquiry messages can be increased in proportion to the extent to which the unreceived blood glucose information is determined to exceed the normal range based on the adjacent blood glucose information, or the number or frequency of outputting inquiry messages can be increased when the unreceived blood glucose information is determined to belong to the high-dangerous range based on the adjacent blood glucose information.

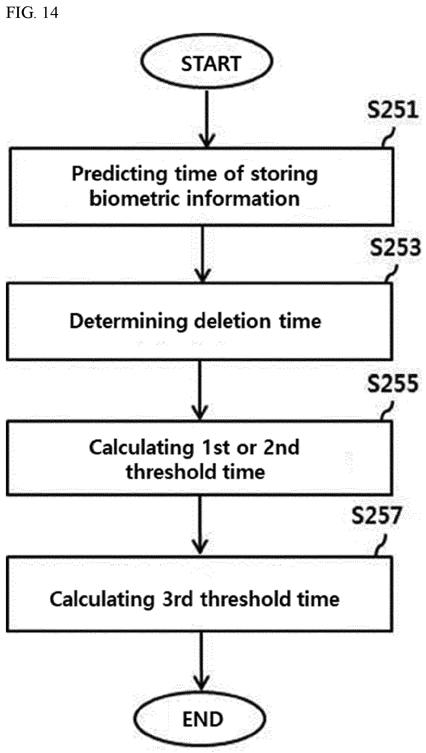

FIG. 14 is a flowchart for illustrating an example of calculating a first threshold time, a second threshold time, or a third threshold time according to an embodiment of the present invention.

Referring to FIG. 14, the sensor transmitter stores blood glucose information measured for a set time after starting to measure blood glucose information, for example, for six (6) hours or twelve (12) hours, in the sensor transmitter, and when a storage period expires, the sensor transmitter sequentially deletes the stored blood glucose information, and, when the unreceived blood glucose information exists, the communication terminal predicts measurement time of the unreceived blood glucose information (S251) and calculates deletion time at which the unreceived blood glucose information is deleted from the sensor transmitter based on the predicted measurement time (S253).

The communication terminal calculates the first threshold time or the second threshold time before the deletion time is reached (S255).

On the other hand, the third threshold time is calculated before the deletion time is reached after the lapse of the second threshold time (S257). Preferably, the sum of the second threshold time and the third threshold time is calculated by being allocated with a ratio set in the total remaining time of from the time when the indicator is output to the deletion time.

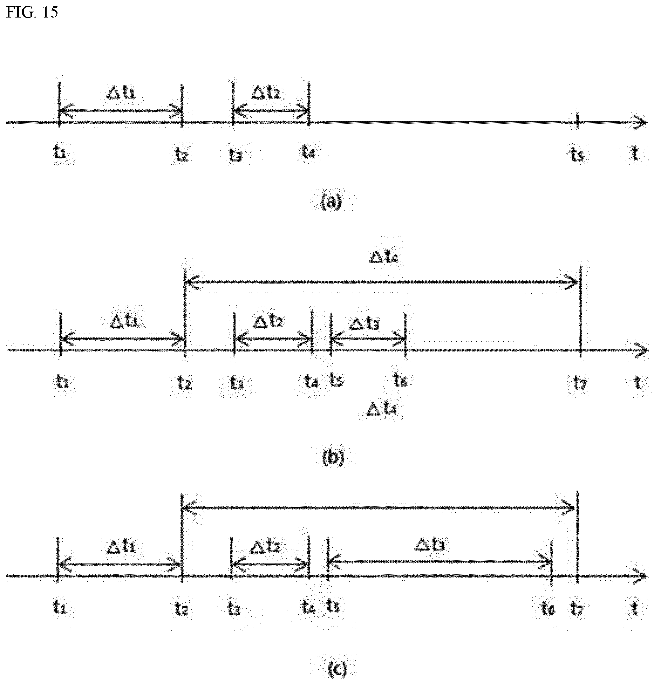

FIG. 15 is a figure for illustrating an example of calculating a first threshold time to a third threshold time, and, as shown in FIG. 15(a), a deletion time (t5) at which unreceived blood glucose information is deleted from the sensor transmitter is calculated based on a time point (T1) at which the existence of unreceived blood glucose information occurs first time. When the sensor transmitter and the communication terminal are connected to each other and start the operation of receiving unreceived blood glucose information, the first threshold time (Δt1) is calculated before the deletion time reaches.

Meanwhile, as shown in FIG. 15(b), a deletion time (t7) at which the unreceived blood glucose information is deleted from the sensor transmitter is calculated based on the time point (T1) at which the existence of the unreceived blood glucose information occurs first time. When the sensor transmitter and the communication terminal are connected to each other and start the operation of receiving unreceived blood glucose information, the second threshold time (Δt2) and the third threshold time (Δt3) are allocated at a certain rate from the total remaining time (Δt4) at which the unreceived blood glucose information is stored to the sensor transmitter, for example, 20% of the total remaining time can be allocated to the second threshold time (Δt2) and 20% of the total remaining time can be allocated to the third threshold time (Δt3). Here, the second threshold time and the third threshold time are allocated to the early part of the total remaining time, so that unreceived blood glucose information can be received or processed as non-receipt without waiting until the total remaining time elapses.

Meanwhile, as shown in FIG. 15(c), a deletion time (t7) at which the unreceived blood glucose information is deleted from the sensor transmitter is calculated based on the time point (T1) at which the existence of the unreceived blood glucose information occurs first time. When the sensor transmitter and the communication terminal are connected to each other and start the operation of receiving unreceived blood glucose information, the second threshold time (Δt2) and the third threshold time (Δt3) are allocated at a certain rate from the total remaining time (Δt4) at which the unreceived blood glucose information is stored to the sensor transmitter, for example, 20% of the total remaining time can be allocated to the second threshold time (Δt2) and the rest of the total remaining time after the lapse of the second threshold time (Δt2) can be allocated to the third threshold time (Δt3). Here, it is characterized in that the third threshold time (Δt3) is allocated in consideration of a communication time set for being capable of connecting communication with the sensor transmitter and receiving the unreceived blood glucose information for the entire remaining time so that when a user command is input within the third threshold time (Δt3) the unreceived blood glucose information can be safely received before the unreceived blood glucose information is deleted from the sensor transmitter.

The present invention described above is implemented through programs executable at computers, and can be operated in a general-purpose digital computer executing the programs using a computer readable medium.

The above-referenced computer readable medium comprises storage medium such as magnetic storage media (e.g., ROM, floppy disks, hard disks, etc.), optical recording media (e.g., CD-ROMs, DVDs, etc.), and carrier waves (e.g., transmission through the Internet).

Although the present invention is described with reference to embodiments shown in the drawings in order to explain the present invention by way of example, a person having ordinary skill in the art which the present invention relates could make various modifications and other embodiments. Accordingly, the protection scope of the present invention shall be defined by the claims attached hereto.

## Claims

1. A computer readable medium containing a program, which, when executed by a computer, causes the computer to carry out a method for transmitting and receiving biometric information between a sensor transmitter (10) configured to be attachable to a body part of a user and measure the biometric information of the user and a communication terminal (30) configured to receive the biometric information from the sensor transmitter (10) and store the received biometric information in a storage module (150), wherein the measured biometric information includes blood glucose information, the method comprising:
determining whether unreceived biometric information among biometric information measured by the sensor transmitter (10) exists based on the biometric information stored in the storage module (150) of the communication terminal (30);
determining whether the unreceived biometric information belongs to a dangerous range or a normal range based on adjacent biometric information, wherein the adjacent biometric information at time adjacent to the unreceived blood glucose information is determined based on a time of measuring the unreceived blood glucose information;
when the unreceived biometric information exists, outputting an indicator for existence of the unreceived biometric information to be informed to the user, wherein information on a range to which the unreceived biometric information belongs is included in the indicator and outputted; and
requesting and receiving the unreceived biometric information from the sensor transmitter (10).

2. The computer readable medium according to claim 1, wherein the method further comprises, when the unreceived biometric information exists, determining a time of measuring the unreceived biometric information,
wherein a range to which the unreceived biometric information belongs is determined based on biometric information received around the time of measuring the unreceived biometric information.

3. The computer readable medium according to claim 1, wherein, when the unreceived biometric information belongs to the normal range, the indicator is processed to be blinded so that the user is unable to visually recognize the indicator.

4. The computer readable medium according to claim 1, wherein, when the unreceived biometric information exists and the unreceived biometric information belongs to the normal range, the unreceived biometric information is requested and received from the sensor transmitter (10) only if a receipt command for receiving the unreceived biometric information is inputted.

5. The computer readable medium according to claim 4, wherein:
when the unreceived biometric information exists and the unreceived biometric information belongs to the normal range, the unreceived biometric information is requested and received from the sensor transmitter (10) if the receipt command for receiving the unreceived biometric information is inputted within a first threshold time, and
when the receipt command is not inputted during the first threshold time, the indicator outputted on a display is deleted.

6. The computer readable medium according to claim 1, wherein the method further comprises:
when the unreceived biometric information exists and the unreceived biometric information belongs to the dangerous range, determining whether the receipt command for receiving the unreceived biometric information is inputted within a second threshold time;
when the receipt command for receiving the unreceived biometric information is not inputted within the second threshold time, outputting to the user an inquiry message for inquiring whether to receive the unreceived biometric information; and
determining whether the receipt command is inputted within a third threshold time after outputting the inquiry message,
wherein:
when the receipt command is inputted within the third threshold time, the unreceived biometric information is requested and received from the sensor transmitter (10), and
when the receipt command is not inputted during the third threshold time, the indicator outputted on a display is automatically deleted.

7. The computer readable medium according to claim 5 or 6, wherein the method further comprises, when the unreceived biometric information exists:
determining time of measuring the unreceived biometric information; and
based on the determined time of measuring the unreceived biometric information, calculating deletion time of deleting the unreceived biometric information of which storage time set after measuring and storing the unreceived biometric information by the sensor transmitter elapses,
wherein at least one of the first threshold time, the second threshold time, and the third threshold time is calculated based on the deletion time.

8. The computer readable medium according to claim 7, wherein the first threshold time, the second threshold time, and the third threshold time are calculated to be prior to the deletion time.

9. The computer readable medium according to claim 1, wherein the method further comprises:
when the unreceived biometric information belongs to the dangerous range, receiving the unreceived biometric information from the sensor transmitter (10) and determining whether the unreceived biometric information belongs to an actual dangerous range; and
when it is determined that the unreceived biometric information belongs to the actual dangerous range, generating and outputting an alarm message notifying the dangerous range to the user.

## Patentansprüche

1. Computerlesbares Medium, das ein Programm enthält, das, wenn es durch einen Computer ausgeführt wird, den Computer veranlasst, ein Verfahren zum Senden und Empfangen biometrischer Informationen zwischen einem Sensorsender (10), der so konfiguriert ist, dass er an einem Körperteil eines Benutzers angebracht werden kann und die biometrischen Informationen des Benutzers misst, und einem Kommunikationsendgerät (30), das so konfiguriert ist, dass es die biometrischen Informationen von dem Sensorsender (10) empfängt und die empfangenen biometrischen Informationen in einem Speichermodul (150) speichert, auszuführen, wobei die gemessenen biometrischen Informationen Blutzuckerinformationen beinhalten, wobei das Verfahren umfasst:
Bestimmen, ob nicht empfangene biometrische Informationen unter den durch den Sensorsender (10) gemessenen biometrischen Informationen vorhanden sind,
basierend auf den in dem Speichermodul (150) des Kommunikationsendgeräts (30) gespeicherten biometrischen Informationen;
Bestimmen, ob die nicht empfangenen biometrischen Informationen zu einem gefährlichen Bereich oder einem normalen Bereich gehören, basierend auf benachbarten biometrischen Informationen, wobei die benachbarten biometrischen Informationen zu einer Zeit benachbart zu den nicht empfangenen Blutzuckerinformationen basierend auf einer Zeit des Messens der nicht empfangenen Blutzuckerinformationen bestimmt werden;
wenn die nicht empfangenen biometrischen Informationen vorhanden sind, Ausgeben eines Indikators für das Vorhandensein der nicht empfangenen biometrischen Informationen zur Information des Benutzers, wobei Informationen über einen Bereich, zu dem die nicht empfangenen biometrischen Informationen gehören, in dem Indikator beinhaltet sind und ausgegeben werden; und
Anfordern und Empfangen der nicht empfangenen biometrischen Informationen von dem Sensorsender (10).

2. Computerlesbares Medium nach Anspruch 1, wobei das Verfahren weiterhin, wenn die nicht empfangenen biometrischen Informationen vorhanden sind, das Bestimmen einer Zeit des Messens der nicht empfangenen biometrischen Informationen umfasst, wobei ein Bereich, zu dem die nicht empfangenen biometrischen Informationen gehören, basierend auf biometrischen Informationen bestimmt wird, die um die Zeit des Messens der nicht empfangenen biometrischen Informationen herum empfangen wurden.

3. Computerlesbares Medium nach Anspruch 1, wobei, wenn die nicht empfangenen biometrischen Informationen zu dem normalen Bereich gehören, der Indikator so verarbeitet wird, dass er ausgeblendet wird, so dass der Benutzer den Indikator visuell nicht erkennen kann.

4. Computerlesbares Medium nach Anspruch 1, wobei, wenn die nicht empfangenen biometrischen Informationen vorhanden sind und die nicht empfangenen biometrischen Informationen zu dem normalen Bereich gehören, die nicht empfangenen biometrischen Informationen nur dann von dem Sensorsender (10) angefordert und empfangen werden, wenn ein Empfangsbefehl zum Empfangen der nicht empfangenen biometrischen Informationen eingegeben wird.

5. Computerlesbares Medium nach Anspruch 4, wobei:
wenn die nicht empfangenen biometrischen Informationen vorhanden sind und die nicht empfangenen biometrischen Informationen zu dem normalen Bereich gehören, die nicht empfangenen biometrischen Informationen von dem Sensorsender (10) angefordert und empfangen werden, wenn der Empfangsbefehl zum Empfangen der nicht empfangenen biometrischen Informationen innerhalb einer ersten Schwellenzeit eingegeben wird, und
wenn der Empfangsbefehl während der ersten Schwellenzeit nicht eingegeben wird, der auf einem Display ausgegebene Indikator gelöscht wird.

6. Computerlesbares Medium nach Anspruch 1, wobei das Verfahren weiterhin umfasst:
wenn die nicht empfangenen biometrischen Informationen vorhanden sind und die nicht empfangenen biometrischen Informationen zu dem gefährlichen Bereich gehören, Bestimmen, ob der Empfangsbefehl zum Empfangen der nicht empfangenen biometrischen Informationen innerhalb einer zweiten Schwellenzeit eingegeben wird; wenn der Empfangsbefehl zum Empfangen der nicht empfangenen biometrischen Informationen nicht innerhalb einer zweiten Schwellenzeit eingegeben wird, Ausgeben einer Anfragenachricht an den Benutzer, um anzufragen, ob die nicht empfangenen biometrischen Informationen empfangen werden sollen; und
Bestimmen, ob der Empfangsbefehl innerhalb einer dritten Schwellenzeit nach dem Ausgeben der Anfragenachricht eingegeben wird,
wobei:
wenn der Empfangsbefehl innerhalb der dritten Schwellenzeit eingegeben wird, die nicht empfangenen biometrischen Informationen von dem Sensorsender (10) angefordert und empfangen werden, und
wenn der Empfangsbefehl nicht während der dritten Schwellenzeit eingegeben wird, der auf einem Display ausgegebene Indikator automatisch gelöscht wird.

7. Computerlesbares Medium nach Anspruch 5 oder 6, wobei das Verfahren weiterhin umfasst, wenn die nicht empfangenen biometrischen Informationen vorhanden sind:
Bestimmen der Zeit des Messens der nicht empfangenen biometrischen Informationen; und
basierend auf der bestimmten Zeit des Messens der nicht empfangenen biometrischen Informationen, Berechnen der Löschzeit des Löschens der nicht empfangenen biometrischen Informationen, deren Speicherzeit, die nach dem Messen und Speichern der nicht empfangenen biometrischen Informationen durch den Sensorsender eingestellt wurde, abläuft,
wobei zumindest eine der ersten Schwellenzeit, der zweiten Schwellenzeit und der dritten Schwellenzeit basierend auf der Löschzeit berechnet wird.

8. Computerlesbares Medium nach Anspruch 7, wobei die erste Schwellenzeit, die zweite Schwellenzeit und die dritte Schwellenzeit so berechnet werden, dass sie vor der Löschzeit liegen.

9. Computerlesbares Medium nach Anspruch 1, wobei das Verfahren weiterhin umfasst:
wenn die nicht empfangenen biometrischen Informationen zu dem gefährlichen Bereich gehören, Empfangen der nicht empfangenen biometrischen Informationen von dem Sensorsender (10) und Bestimmen, ob die nicht empfangenen biometrischen Informationen zu einem tatsächlichen gefährlichen Bereich gehören; und
wenn bestimmt wird, dass die nicht empfangenen biometrischen Informationen zu dem tatsächlichen gefährlichen Bereich gehören, Erzeugen und Ausgeben einer Alarmnachricht, die dem Benutzer den gefährlichen Bereich meldet.

## Revendications

1. Support lisible par ordinateur contenant un programme dont l'exécution par un ordinateur entraîne la mise en œuvre par ledit ordinateur d'un procédé de transmission et de réception d'informations biométriques entre un émetteur de détection (10), prévu pour pouvoir être fixé sur une partie du corps d'un utilisateur et mesurer les informations biométriques de l'utilisateur, et un terminal de communication (30) prévu pour recevoir les informations biométriques provenant de l'émetteur de détection (10) et stocker les informations biométriques reçues dans un module de mémorisation (150), les informations biométriques mesurées comprenant des informations relatives à la glycémie, ledit procédé comprenant :
la détermination si des informations biométriques non reçues parmi les informations biométriques mesurées par l'émetteur de détection (10) sont présentées sur la base des informations biométriques stockées dans le module de mémorisation (150) du terminal de communication (30);
la détermination si les informations biométriques non reçues appartiennent à une plage dangereuse ou à une plage normale sur la base d'informations biométriques adjacentes, les informations biométriques adjacentes au moment adjacent aux informations relatives à la glycémie non reçues étant déterminées sur la base d'un temps de mesure des informations relatives à la glycémie non reçues;
si les informations biométriques non reçues sont présentées, l'émission d'un indicateur d'existence des informations biométriques non reçues à notifier à l'utilisateur, les informations sur une plage à laquelle appartiennent les informations biométriques non reçues étant comprises dans l'indicateur et émises; et
la demande et la réception de l'émetteur de détection (10) des informations biométriques non reçues.

2. Support lisible par ordinateur selon la revendication 1, où le procédé comprend en outre, si les informations biométriques non reçues sont présentées, la détermination d'un moment de mesure des informations biométriques non reçues,
où une plage à laquelle appartiennent les informations biométriques non reçues est déterminée sur la base des informations biométriques reçues autour du moment de mesure des informations biométriques non reçues.

3. Support lisible par ordinateur selon la revendication 1, où, si les informations biométriques non reçues appartiennent à la plage normale, l'indicateur est traité pour être masqué de manière à rendre l'utilisateur inapte à reconnaître visuellement l'indicateur.

4. Support lisible par ordinateur selon la revendication 1, où, si les informations biométriques non reçues sont présentées et appartiennent à la plage normale, les informations biométriques non reçues sont demandées et reçues de l'émetteur de détection (10) seulement si une instruction de réception pour recevoir les informations biométriques non reçues est entrée.

5. Support lisible par ordinateur selon la revendication 4, où :
si les informations biométriques non reçues sont présentées et que les informations biométriques non reçues appartiennent à la plage normale, les informations biométriques non reçues sont demandées et reçues de l'émetteur de détection (10) si l'instruction de réception pour recevoir les informations biométriques non reçues est entrée dans un premier délai seuil, et
si l'instruction de réception n'est pas entrée dans le premier délai seuil, l'indicateur émis sur un écran est supprimé.

6. Support lisible par ordinateur selon la revendication 1, où le procédé comprend en outre :
si les informations biométriques non reçues sont présentées et que les informations biométriques non reçues appartiennent à la plage dangereuse, la détermination si l'instruction de réception pour recevoir les informations biométriques non reçues est entrée dans un deuxième délai seuil ;
si l'instruction de réception pour recevoir les informations biométriques non reçues n'est pas entrée dans le deuxième délai seuil, l'émission à destination de l'utilisateur d'un message d'interrogation s'il doit recevoir les informations biométriques non reçues ; et
la détermination si l'instruction de réception est entrée dans un troisième délai seuil après l'émission du message d'interrogation,
où :
si l'instruction de réception est entrée dans le troisième délai seuil, les informations biométriques non reçues sont demandées et reçues par l'émetteur de détection (10), et,
si l'instruction de réception n'est pas entrée dans le troisième délai seuil, l'indicateur émis sur un écran est automatiquement supprimé.

7. Support lisible par ordinateur selon la revendication 5 ou la revendication 6, où le procédé comprend en outre, si les informations biométriques non reçues sont présentées :
la détermination du temps de mesure des informations biométriques non reçues ; et,
sur la base du temps déterminé de mesure des informations biométriques non reçues, le calcul du temps de suppression des informations biométriques non reçues dont le temps de stockage défini après la mesure et le stockage des informations biométriques non reçues par l'émetteur de détection est écoulé,
où le premier délai seuil et/ou le deuxième délai seuil et/ou le troisième délai seuil sont calculés sur la base du temps de suppression.

8. Support lisible par ordinateur selon la revendication 7, où le premier délai seuil, le deuxième délai seuil et le troisième délai seuil sont calculés antérieurs au temps de suppression.

9. Support lisible par ordinateur selon la revendication 1, où le procédé comprend en outre :
si les informations biométriques non reçues appartiennent à la plage dangereuse, la réception des informations biométriques non reçues de l'émetteur de détection (10) et la détermination si les informations biométriques non reçues appartiennent à une plage dangereuse effective ;
et,
s'il est déterminé que les informations biométriques non reçues appartiennent à la plage dangereuse effective, la génération et l'émission d'un message d'alerte notifiant la plage dangereuse à l'utilisateur.
